# EUROPEAN PATENT SPECIFICATION

(11) **EP 3 630 103 B1**
(45) Date of publication and mention of the grant of the patent: **30.10.2024**
(21) Application number: 18806042.0
(22) Date of filing: 24.05.2018
(51) Int. Cl.: A61K 31/42, A61K 31/55, A61K 31/06, A61K 31/135, A61K 31/495, A61K 31/381, A61P 25/24, A61P 25/00, A61K 9/00, A61K 31/165, A61K 31/4152, A61K 31/4525, A61K 31/554, A61K 45/06, A61K 47/40, A61P 25/22

(54) **COMBINED THERAPY FOR NMDAR ANTAGONIST-RESPONSIVE NEUROPSYCHIATRIC DISORDERS**
KOMBINATIONSTHERAPIE FÜR AUF NMDAR-ANTAGONIST ANSPRECHENDE NEUROPSYCHIATRISCHE ERKRANKUNGEN
TRAITEMENT COMBINÉ DES TROUBLES NEUROPSYCHIATRIQUES SENSIBLES AUX ANTAGONISTES DU NMDAR

(30) Priority: 25.05.2017 US 201762510801 P; 12.06.2017 US 201762518020 P
(43) Date of publication of application: 08.04.2020
(73) Proprietor: Glytech LLC., Dobbs Ferry, NY 10522 (US)
(72) Inventor: JAVITT, Daniel C., 9318711 Jerusalem (IL)
(74) Representative: Schulz Junghans Patentanwälte PartGmbB
(86) International application number: PCT/IL2018/050570
(87) International publication number: WO 2018/216020

(56) References cited:
- WO-A1-2012/104852
- WO-A2-2014/011590
- JOSEPH R MOSKAL ET AL: "GLYX-13, an NMDA receptor glycine site functional partial agonist enhances cognition and produces antidepressant effects without the psychotomimetic side effects of NMDA receptor antagonists", EXPERT OPINION ON INVESTIGATIONAL DRUGS, vol. 23, no. 2, 20 November 2013 (2013-11-20), UK, pages 243 - 254, XP055422742, ISSN: 1354-3784, DOI: 10.1517/13543784.2014.852536

## Description

### CROSS REFERENCE TO RELATED APPLICATIONS

Benefit is claimed to US Provisional Patent Application No. 62/510,801, filed May 25, 2017, and US Provisional Patent Application No. 62/518,020, filed June 12, 2017.

### FIELD

Provided herein is a composition for use in treatment of a NMDA receptor neuropsychiatric disorder selected from the group consisting of obsessive compulsive disorder and anxiety disorders in a human subject in need thereof, said composition comprising a therapeutic effective amount of a first agent consisting of D-cycloserine, and a second agent consisting of S-mirtazapine, as further defined in the claims.

The invention is defined in the claims. Any subject-matter outside the scope of the claims does not form part of the invention and is provided for information and/or comparison purposes only.

Any reference to a method of treatment of the human or animal body by therapy involving a compound or composition is to be interpreted as a reference to said compound or composition for use in said treatment.

### BACKGROUND

Major depression is a clinical syndrome that includes a persistent sad mood or loss of interest in activities, which persists for at least two weeks in the absence of treatment. Symptoms of major depression are typically measured using rating scales such as the Hamilton Depression Rating Scale (HAM-D) or the Beck Depression Inventory (BDI). In addition to including symptoms relevant to depressed mood, the HAM-D also contains symptoms sensitive to psychosis, including items for guilt, depersonalization/derealization and paranoia. Major depression may also be associated with symptoms of anxiety, which may be measured with rating scales such as the Hamilton Rating Scale for Anxiety (HAM-A). Depressive disorders are divided into major depression (MDD) and bipolar depression (BPD). Major depression may also occur with and without melancholic features. In addition, depressive symptoms may occur in the context of anxiety disorders such as generalized anxiety disorder, dissociative disorders, personality disorders or adjustment disorders with depressed mood (DSM-IV).

Other forms of depression include atypical depression, agitated depression, depression with mixed emotional features, cyclothymia, dysthymia minor depression and adjustment disorder with depressed mood. Bipolar depression may be divided into Bipolar I and Bipolar II subtypes based upon presence or absence of manic episodes. In bipolar disorder, depressive symptoms can occur in the context of either a depressive episode, or a mixed state in which symptoms of mania and depression occur simultaneously or in rapid sequence. Rapid cycling between mania and depressive episodes may also occur in some individuals.

Risk for suicide is significantly increased in depressive disorders, but may respond differentially to medication versus depressive symptoms as a whole. When suicide occurs, it is often accompanied by feelings of worthlessness or inappropriate guilt, as well as recurrent thoughts of death or suicidal ideation and guilt is an accepted proxy for suicide. While the risk of suicide increases in subjects with a depressive disorder, medications used to date to typically treat depressive disorders paradoxically increase suicidal tendencies.

Most current theories of depression focus on serotonergic and/or noradrenergic brain systems. Current treatments for major depression consist primarily of older antidepressants, such as monoamine oxidase inhibitors (MAOI) and tricyclic antidepressants (TCAs) (e.g. imipramine, amitryptiline, desipramine, clomipramine) that were first developed in the 1960's, and newer agents such as tetracyclic antidepressants (TeCAs) (e.g., mianserin, mirtazapine), serotonin (SSRI) and serotonin/norephinephrine (SNRI) reuptake inhibitors (e.g., fluoxetine, fluvoxamine, paroxetine, citalopram, escitalopram, duloxetine, venlafaxine, dapoxetine, indalpine, milnacipran, levomilnacipran). MAOIs and TCAs are considered "broader spectrum" agents than SSRIs/SNRIs that were developed subsequently.

Glutamate is an alternative brain neurotransmitter that has been studied to a limited degree in relationship to depression or other affective disorders. Glutamate binds to several receptor types including N-methyl-D-aspartate type glutamate receptors (NMDAR). NMDAR contain multiple binding sites including an agonist site for glutamate and an allosteric modulatory site (aka glycineB receptor, strychnine-insensitive glycine receptor) sensitive to the endogenous brain amino acids glycine and D-serine. Agonists at the glycine site increase NMDAR activation in response to glutamate while antagonists decrease NMDAR activation.

Functional agonists and antagonists at the glycine site can be identified using well-validated electrophysiological assays such as modulation of NMDAR-mediated responses to NMDAR glutamate-site agonists, or radio-receptor assays, such as modulation of binding to the NMDAR PCP-receptor channel binding site. Glycine site agonists and antagonists can also be distinguished based upon both electrophysiology and receptor binding from compounds such as phencyclidine (PCP) or ketamine that bind to the channel site (aka PCP receptor, uncompetitive antagonist site) of the NMDAR. Effective agonists and antagonists may be identified, for example, as compounds with <100 nM affinity for their target and >10-fold selectivity vs. other relevant targets. Partial agonists are defined as compounds that have reduced efficacy for inducing conformational change in receptors (typically 40-80%) relative to full agonists, and which may induce agonist effects at low dose but antagonist effects at high dose.

Despite the wide range of pharmacological options, current treatment approaches for depression have severe limitations. Only 60-65% of patients respond to the initial regimen and among those responding, less than half either reach remission or become symptom-free. Individuals not responding to a first course of antidepressant treatment are often switched to a different drug, with results that are generally modest and incremental.

Within major depression, treatment-refractory depression refers to a form of depression that responds poorly to currently available treatments (e.g., *see* nimh.nih.gov/trials/practical/stard/index.shtml June 2011) and which may have different underlying etiopathological mechanisms compared with other forms of depression. Combinations of antidepressants have not been shown to be superior to monotherapy for refractory depression and typically increase risk of side effects and are not recommended. Thus a continuing need exists for effective treatments for major depression and other disorders related to excess NMDAR activation.

### SUMMARY

Newer SNRIs and atypical antidepressants differ from traditional medications in that they have higher specificity for targets other than the serotonin transporter, and thus are relatively more similar to TCAs in having balanced modulation of serotonergic and noradrenergic symptoms. In general, the newer agents have efficacy similar to those of more traditional SSRIs and SNRIs, but differential side effect profiles (Wagner et al., Journal of Affective Disorders 228:1-12, 2018). Their relative interaction with ketamine or other NMDAR-based antidepression agents has not been previously studied. It has now been observed unexpectedly that newer SNRIs/atypical antipsychotics have preferential beneficial activities in combination with a D-cycloserine-dose associated with plasma levels >25 microgram/mL, and thus show unexpected utility of combinations involving high dose D-cycloserine and newer anti-depressant agents.

Accordingly, described herein are formulations containing a NMDAR antagonistcombined with an anti-depressant that is drawn from a list that includes levomilnacipran, milnacipran, vilazadone, vortioxetine, S-mirtazapine and R-mirtazapine.

Uses of the described compositions in methods for treatment of depression, OCD, and anxiety disorders, and for preparation of a medicament for use in such treatments are also described.

The invention is defined in the claims.

WO 2012/104852 A1 discloses that the compound D-cycloserine can be used as an antidepressant compound. It is further disclosed that a combination with newer antidepressants, including in particular tetracylic antidepressants and SSRIs/SNRIs, can be used advantageously to prevent psychotic reactions which can be caused by D-cycloserine if used at high doses.

The foregoing and other objects, features, and advantages will become more apparent from the following detailed description, which proceeds with reference to the accompanying figures.

### BRIEF DESCRIPTION OF THE DRAWINGS

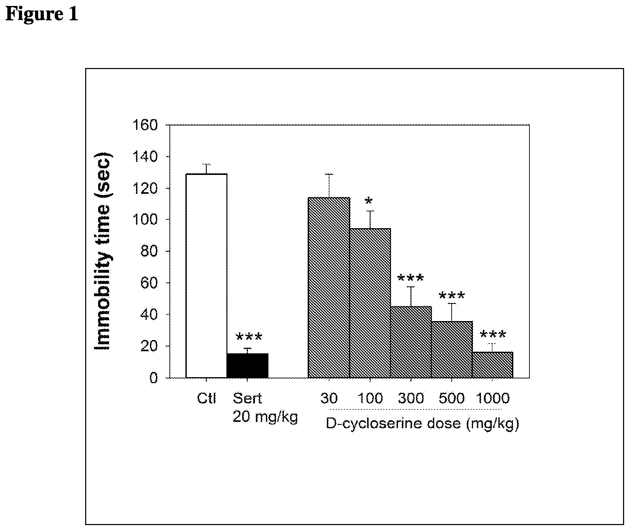

**Figure 1** is a graph showing the effect of indicated mg/kg (mpk) doses of D-cycloserine (DCS) on immobility time in the forced swim test (FST) following oral (PO) administration relative to control (ctl) or active comparator (sertraline). * p<.05 vs. Ctl; ***p<.001 vs. Ctl

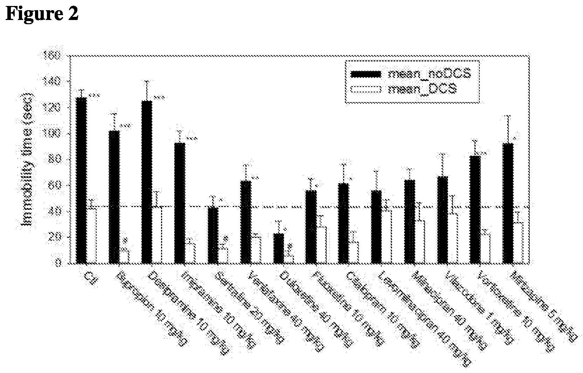

**Figure 2** is a graph showing the effect of DCS in combination with indicated antidepression agents. As indicted, *p<0.05, **p<0.01, ***p<0.001 No DCS vs. 300 mg/kg. #p<0.05 vs. DCS 300 mg/kg alone.

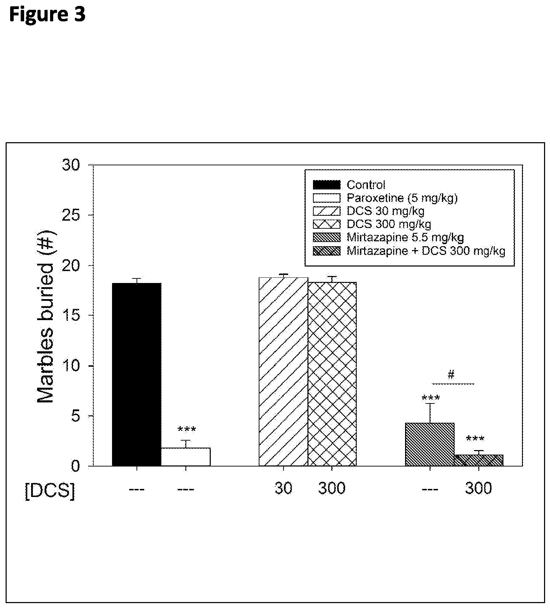

**Figure 3** shows the synergistic effects of DCS and mirtazapine on marble burying ***p<0.001 vs. Vehicle. #p<0.05 mirtazapine + DCS 300 mg/kg vs. mirtazapine alone. 10 mice were treated with either Vehicle (control), Paroxetine(5 mg/kg), Mirtazapine (5.5 mg/kg), D-cycloserine (30 mg/kg), D-cycloserine (300 mg/kg) or D-cycloserine (300 mg/kg) + (Mirtazapine 5.5 mg/kg) which was administered by IP 30 minutes prior to test. Distance traveled during the test was captured by cameras and quantified using Video Tracker Software (ViewPoint Life Sciences Software, France). At the end of the test mice were removed from the cages and the number of unburied marbles was counted. A marble was considered buried if it was covered at least two thirds with bedding. An effect was considered significant if p < 0.05.

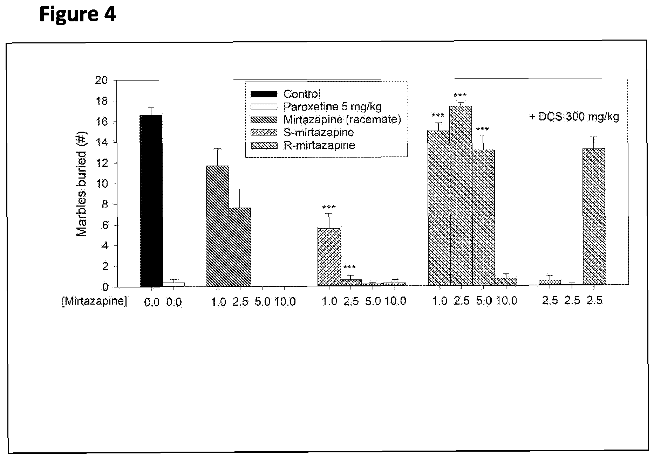

**Figure 4** shows the relative effects of R- and S-isomers of mirtazapine on marble burying, showing greater effects of R- vs S-isomer. ***p<0.001 vs. racemic mirtazapine. 10 mice were treated with either Vehicle (control), Paroxetine (5 mg/kg), S-Mirtazapine (1, 2.5, 5.0, and 10 mg/kg), R-Mirtazapine (1, 2.5, 5.0, and 10 mg/kg), R-Mirtazapine (2.5mg/kg)+ D-cycloserine (300 mg/kg) which was administered by IP 30 minutes prior to test. Distance traveled during the test was captured by cameras and quantified using Video Tracker Software (ViewPoint Life Sciences Software, France). At the end of the test mice were removed from the cages and the number of unburied marbles was counted. A marble was considered buried if it was covered at least two thirds with bedding. An effect was considered significant if p < 0.05.

### DETAILED DESCRIPTION

### I. Terms

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." "Consisting essentially of" indicates a composition, method, or process that includes only those listed features as the active or essential elements, but can include non-active elements in addition. The abbreviation, *"e.g."* is derived from the Latin *exempli gratia,* and is used herein to indicate a non-limiting example. Thus, the abbreviation "e.g." is synonymous with the term "for example."

In case of conflict, the present specification, including explanations of terms, will control. In addition, all the materials, methods, and examples are illustrative.

**Administration:** The introduction of a composition into a subject by a chosen route. Administration of an active compound or composition can be by any route known to one of skill in the art. Administration can be local or systemic. Systemic administration includes any route of administration designed to distribute an active compound or composition widely throughout the body via the circulatory system. Thus, systemic administration includes, but is not limited to oral, intra-arterial and intravenous administration.

**Antagonist:** A molecule or compound that tends to nullify the action of another, or in some instances that blocks the ability of a given chemical to bind to its receptor or other interacting molecule, preventing a biological response.

**Effective amount** or a **therapeutically effective amount:** indicates a nontoxic but sufficient amount of the same to provide the desired effect. In a combination therapy of the present invention, an "effective amount" of one component of the combination is the amount of that compound that is effective to provide the desired effect when used in combination with the other components of the combination. The amount that is "effective" will vary from subject to subject, depending on the age and general condition of the individual, the particular active agent or agents, and the like. Thus, it is not always possible to specify an exact "effective amount." However, an appropriate "effective" amount in any individual case may be determined by one of ordinary skill in the art using routine experimentation.

**D-cycloserine (DCS):** refers to the chemical D-cycloserine (CA Index Name: 3-Isoxazolidinone, 4-amino-, (4R)- (9CI); CAS Registry No. 68-41-7), or pharmaceutically acceptable salts thereof. DCS is an FDA (United States Food and Drug Administration)-approved drug for treatment of tuberculosis, and is sold by Eli Lilly and Company under the trade name Seromycin^{®}. DCS is a structural analog of D-alanine, and is a broad-spectrum antibiotic produced by some strains of *Streptomyces orchidaceus* and *S*. *garphalus.* DCS is also described herein as "cycloserine."

**Parenteral:** Administered outside of the intestine, for example, not via the alimentary tract. Generally, parenteral formulations are those that will be administered through any possible mode except ingestion. This term especially refers to injections, whether administered intravenously, intrathecally, intramuscularly, intraperitoneally, or subcutaneously, and various surface applications including intranasal, intradermal, and topical application, for instance.

**Pharmaceutically acceptable carriers:** The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the compounds herein disclosed. In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Pharmaceutical agent:** A chemical compound or composition capable of inducing a desired therapeutic or prophylactic effect when properly administered to a subject or a cell.

**Subject:** Living multi-cellular organisms, including vertebrate organisms, a category that includes both human and non-human mammals. The human subjects described herein are also referred to as "patients."

**Subject susceptible to a disease or condition:** A subject capable of, prone to, or predisposed to developing a disease or condition. It is understood that a subject already having or showing symptoms of a disease or condition is considered "susceptible" since they have already developed it. For example, it is appreciated that patients suffering from depression are susceptible to suicidality.

**Treating** and **Treatment:** refer to reduction in severity and/or frequency of symptoms, elimination of symptoms and/or their underlying cause, prevention of the occurrence of symptoms and/or their underlying cause, and improvement or remediation of damage. Thus, for example, "treating" a patient involves prevention of a particular disorder or adverse physiological event in a susceptible individual as well as treatment of a clinically symptomatic individual.

### II. Overview of Several Embodiments

Described herein are compositions for use in treatment of a NMDA receptor neuropsychiatric disorder in a human subject, which includes a therapeutic effective amount of a first agent that is a NMDA receptor antagonist; and a second agent that is an anti-depressant selected from levomilnacipran, milnacipran, vilazadone, vortioxetine, S-mirtazapine and R-mirtazapine, and wherein the NMDA receptor neuropsychiatric disorder is selected from depression, obsessive compulsive disorder, and anxiety disorders The invention concerns the treatment of NMDA receptor neuropsychiatric disorders selected from the group consisting of obsessive compulsive disorder and anxiety disorders.

According to the invention, the first agent is D-cycloserine provided at a net antagonistic dose of ≥500 mg/day to ≤1000 mg/day and is formulated to produce an average plasma level in the subject of greater than 25 µg/mL, and more particularly a dose of 7.5-12.5 mg/kg/day.

According to the invention, the second agent is the antidepressant S-mirtazapine.

In still further embodiments, the described composition is formulated in a sustained release formulation.

Methods of treatment of a NMDA receptor-responsive neuropsychiatric disorder selected from depression, obsessive compulsive disorder, and anxiety disorders, by administering to a subject a therapeutically effective amount of any of the above described compositions are also described.

Additionally provided are uses of the described compositions in the preparation of a medicament for treatment of the described conditions.

### III. Methods for treatment of Depression, OCD, and Anxiety Disorders

Demonstrated herein is the finding that antagonistic D-cycloserine plasma levels are associated with rodent anti-depressive effects. Also provided is the first demonstration of D-cycloserine augmentation of locomotor hyperactivity, and the first demonstration that different classes of antidepressant are differentially capable of modulating locomotor hyperactivity in combination with D-cycloserine provided at a net antagonist dosage, suggesting differential clinical effectiveness. These findings permit development of unexpected dosing strategies for D-cycloserine for the treatment of human neuropsychiatric illness, and suggest unexpected beneficial combinatorial effects of D-cycloserine with specific types of anti-depressant medication, and particularly, more recently developed antidepressant agents. In view of these and other described observations, provided herein are compositions and methods for treatment of neuropsychiatric conditions associated with excessive NMDR neurotransmission, and particularly depression, obsessive-compulsive disorder, and anxiety disorders.

The compositions described herein are composed of an NMDAR antagonist compound. NMDARs are a type of neuronal receptor for the brain neurotransmitter glutamate. NMDARs participate in a range of brain functions including sensory processing, cognition, and emotion regulation. NMDARs are comprised of multiple subunits termed GluN1, GluN2 and GluN3 (formerly NR1, NR2, NR3). Multiple forms of GluN1, GluN2 and GluN3 exist. An NMDAR may consist of various combinations of GluN1, GluN2 and GluN3 subunits in various amounts. Agonists and antagonists may affect all NMDARs equivalently, or may be selective for an NMDAR containing specific subunit types. The methods described herein include use of NMDAR antagonists.

Recently, the non-competitive NMDAR antagonist ketamine has also been shown to have antidepressant effects in humans when tested in individuals with treatment-resistant depression. The compound shows similar effects in both unipolar and bipolar depression. Other non-competitive NMDAR antagonists such as MK-801 also show anti-depressant effects in animal models. However, antidepressant effects induced by ketamine are associated with exacerbation of psychosis, which greatly reduces their utility in clinical situations.

NMDARs contain binding sites for the neurotransmitter glutamate and for the endogenous modulatory amino acids glycine and D-serine. NMDARs also are sensitive to the redox state of the surrounding tissue via a redox site/polyamine binding site. Agents that bind to these sites and reduce NMDAR activity are termed competitive inhibitors.

The NMDAR glutamate binding site selectively binds the synthetic glutamate derivative N-methyl-D-aspartate with high affinity. This site is alternately referred to as the glutamate recognition site or the NMDA recognition site of the NMDAR.

The NMDAR glycine/D-serine binding site has been referred to as the glycine modulatory site, the allosteric modulatory site or the glycine-B receptor.

NMDARs form an ion channel that is blocked by several drugs of abuse, such as phencyclidine (PCP), ketamine, or dizocilpine (MK-801). These compounds bind to a site that has been termed the PCP receptor. Agents that block the NMDAR-associated ion channel are collectively termed non-competitive NMDAR antagonists, or NMDAR channel blockers. Blockade of NMDARs by channel blockers leads to a clinical psychotic state that closely resembles schizophrenia.

In the described methods, NMDARs may also be inhibited by antagonists that bind to the glutamate recognition sites, the glycine recognition site, or the polyamine binding site. Historically, high affinity NMDAR antagonists have been used in multiple clinical settings.

Selfotel (CGS 19755) is an example of an antagonist that binds to the glutamate recognition site. Several such compounds were developed for CNS indications such as stroke or epilepsy. When used at doses sufficient to significantly inhibit NMDAR, these compounds, like channel blockers, lead to clinical psychotomimetic symptoms.

Additional compounds that function as antagonists of the glutamate recognition site include aptiganel (Cerestat, CNS-1102) and related compounds as described in Reddy et al., J Med Chem 37:260-7. 1994). Additional compounds that function as antagonists of the glutamate recognition site include alpha.-amino-carboxylic acid and phosphonic acid functionalities separated by a variety of spacer units. An unembellished example is 2-amino-5-phosphonovaleric acid (AP5) (Watkins, J. C.; Evans, R. H., Annu. Rev. Pharmacol. Toxicol. 1981, 21, 165), which contains a saturated carbon chain. More complex examples, which contain elements enhancing structural rigidity and therefore potency, include CPP, cis-4-(phosphonomethyl)-2-piperidinecarboxylic acid (CGS-19755) (Lehman, J. et al., J. Pharmacol. Exp. Ther. 1988, 246, 65), and (E)-2-amino-4-methyl-5-phosphono-3-pentenoic acid (CGP-37849) (Schmutz, M. et al., Abs. Soc. Neurosci. 1988, 14, 864). See U.S. Pat. No. 7,345,032, issued Mar 18, 2008 and U.S. Pat. No 5,168,103.

In the described methods, NMDARs may also be inhibited by antagonists that bind to the glycine recognition site. In a particular embodiment, such inhibition is by D-cycloserine, administered at an antagonist producing dosage.

D-cycloserine, also referred to herein as "cycloserine", is a compound currently approved for treatment of tuberculosis (TB). Psychotropic effects of cycloserine were noted in the late 1950's in patients being treated for TB. In an initial report, effects of cycloserine were noted on symptoms such as anorexia, asthenia and insominia. However, no formal psychiatric diagnoses were made. Furthermore, cycloserine was recommended primarily for treatments of tension and insomnia, as opposed to depression.

Because of its ability to bind to NMDAR and because of theories linking NMDAR to schizophrenia, D-cycloserine has been studied in treatment resistant schizophrenia. At low doses, D-cycloserine has been found to produce beneficial effects in some but not all studies, and may exacerbate symptoms in individuals receiving clozapine. Furthermore, at higher doses (>250 mg), however, D-cycloserine exacerbates psychosis and so according to package label insert is contra-indicated in schizophrenia, depression and anxiety disorders. Research with D-cycloserine in preclinical models has also not suggested its usefulness at high dose in treatment of depression. Partial agonists of NMDAR, in particular 1-aminocyclopropanecarboxylic acid (ACPC) have been reported to have efficacy in animal models, but have not yet been tested in human studies. Furthermore, effects were only observed at the lowest dose tested, arguing away from high dose treatment in humans. In animal depression models, tolerance over weeks has also been observed, arguing against sustained long term use.

For example, D-cycloserine reported for use at a dose of 250 mg/day was found to be without significant effect on symptoms of major depression and moreover, commonly available prescribing information states that D-cycloserine use is contraindicated in individuals with a history of epilepsy, depression, severe anxiety, or psychosis (Lilly. Seromycin (cycloserine) capsules prescribing information. Indianapolis, Ind.; 2005 Apr. 28).

D-cycloserine produces primarily agonist effects at doses up to approximately 100 mg, and primarily antagonist effects at doses above 500 mg, with intermediate effects at intermediate doses. Plasma concentrations associated with primarily agonist effects are primarily <10 µg/mL. Plasma concentrations associated with antagonist effects are >25 µg/ml. Increased liability for toxicity is observed at plasma levels >35 µg/mL.

D-cycloserine is typically administered for the treatment of tuberculosis at doses of 250-1000 mg. Thus, typical doses are 250 mg, 500 mg, 750 mg or 1000 mg. Intermediate doses, such as 550, 600, 650, 700, 800, 850 or 900 mg are also possible. According to the invention, D-cycloserine is administered to a subject at a dose of greater than 500 mg/day to less than or equal to 1000 mg/day, including but not limited to the above intermediate doses. Effective doses of D-cycloserine for the intended use in humans require sustained plasma levels exceed >25 ug/ml, which in particular embodiments is provided by a dosage of greater than or equal to10 mg/kg in an adult subject. Achievement of these levels requires human doses in excess of 500 mg/day, which in an average adult will be about 700 mg/day or greater. Human dosing of D-cycloserine to produce net antagonist effects can be understood from human pharmacokinetics studies.

Pharmacokinetics (PK) of D-cycloserine in humans after a dose of 500 mg have been previously studied. Critical parameters include maximum (peak) concentration achieved (Cmax), time to maximum concentration (Tmax) and area under the curve (AUC) during the dosing interval.

For example, Zhu et al. (Zhu M, Nix DE, Adam RD, Childs JM, Peloquin CA. Pharmacokinetics of cycloserine under fasting conditions and with high-fat meal, orange juice, and antacids. Pharmacotherapy. 2001;21(8):891-7) showed median Cmax values of 14.8 microgram/mL under fasting conditions, with a range of 12.1-30.6 microgram/mL. Median AUC levels over 24 hr were 214 microgram-hr/mL with a range of 163-352, corresponding to median sustained plasma levels of 8.9 microgram/mL with a range of 6.8 - 14.7 microgram/mL.

Park et al., (Park SI, Oh J, Jang K, Yoon J, Moon SJ, Park JS, Lee JH, Song J, Jang IJ, Yu KS, Chung JY. Pharmacokinetics of Second-Line Antituberculosis Drugs after Multiple Administrations in Healthy Volunteers. Antimicrob Agents Chemother. 2015;59(8):4429-35.) evaluated pharmacokinetics of 250 mg PO D-cycloserine given every 12 hrs, and observed mean Cmax values of 24.9 microgram/mL and a mean AUC over 12 hrs of 242.3 mg-h/L, corresponding to a mean plasma level of 20. 2 microgram/mL.

Hung et al., 2014 (Hung WY, Yu MC, Chiang YC, Chang JH, Chiang CY, Chang CC, Chuang HC, Bai KJ. Serum concentrations of cycloserine and outcome of multidrug-resistant tuberculosis in Northern Taiwan. Int J Tuberc Lung Dis. 2014;18(5):601-6) evaluated PK levels during clinical treatment with DCS. Mean dose across subjects was 8.8 mg/kg, with the majority of subjects (n=27) receiving 500 mg/day DCS, and a minority either 750 mg/d (n=4) or 250 mg/d (n=2). DCS concentrations at 2 and 6 hr after dosing were 19.7 and 18.1 microgram/mL.

Thus, a consistent finding of human PK studies is that sustained plasma doses following 500 mg administration of D-cycloserine are consistently below 25 microgram/mL. As described herein, the antidepressant effects of D-cycloserine are observed at dosages above 25 microgram/mL. Accordingly, the daily dose for producing such plasma levels will necessarily be above 500 mg/day, as described herein. Such doses include amounts greater than 10 mg/kg/day, such as 10, 12, 14, 16, and 18 mg/kg/day.

Felbamate is another example of a compound that may act via the glycine binding site, and which can be used in the described methods. When administered to humans, felbamate produces psychotic effects that limit its clinical utility (e.g. Besag FM, Expert Opin Drug Saf 3:1-8, 2004).

Gavestinel (GV-150,526) is another example of an antagonist at the glycine binding site for use as described herein. Other similarly useful compounds are described in DiFabrio et al., J Med Chem 40:841-50, 1997. Other examples of glycine site antagonists that are suitable for use in the pharmaceutical compositions and methods described herein are those referred to in the following: U.S. Pat. No. 6,667,317 which was issued on Dec. 23, 2003; U.S. Pat. No. 6,080,743 which was issued June 27, 2000; U.S. Pat. No. 5,990,108, which was issued on Nov. 23, 1999; U.S. Pat. No. 5,942,540, which issued on Aug. 24, 1999; World Patent Application WO 99/34790 which issued on Jul. 15, 1999; WO 98/47878, which was published on Oct. 29, 1998; World Patent Application WO 98/42673, which was published on October 1, 1998; European Patent Application EP 966475A1, which was published on Dec. 29, 1991; World Patent Application 98/39327, which was published on Sep. 11, 1998; World Patent Application WO 98/04556, which was published on Feb. 5, 1998; World Patent Application WO 97/37652, which was published on Oct. 16, 1997; U.S. Pat. No. 5,837,705, which was issued on Oct. 9, 1996; World Patent Application WO 97/20553, which was published on Jun. 12, 1997; U.S. Pat. No. 5,886,018, which was issued on Mar. 23, 1999; U.S. Pat. No. 5,801,183, which was issued on Sep. 1, 1998; World Patent Application WO 95/07887, which was issued on Mar. 23, 1995; U.S. Pat. No. 5,686,461, which was issued on Nov. 11, 1997; U.S. Pat. No. 5,622,952, issued Apr. 22, 1997; U.S. Pat. No. 5,614,509, which was issued on Mar. 25, 1997; U.S. Pat. No. 5,510,367, which was issued on Apr. 23, 1996; European Patent Application 517,347A1, which was published on Dec. 9, 1992; U.S. Pat. No. 5,260,324, which published on Nov. 9, 1993.

GlyX-13 (Rapastinel) is a tetra-peptide (threonine-proline-proline-threonine) that functions as a mixed agonist/antagonist at the glycine site. NRX-1074 (apostimel) is an orally available molecule with similar properties to GlyX-13. NYX-2925 ((2S, 3R)-3-hydroxy-2-((R)-5-isobutyryl-1-oxo-2,5-diazaspiro[3.4]octan-2-yl)butanamide) is a small molecule designed based on effects of GlyX-13. AGN-241751 (Allergan) is an orally available, small molecule analog of GlyX-13. CERC-301 (Rislenemdaz) is an orally-active, selective NMDAR subunit 2B antagonist. AZD-6765 (Lanicemine) is a low-trapping NMDAR antagonist. S-ketamine (esketamine) is the S-isomer of racemic ketamine. R-ketamine is the R-isomer of racemic ketamine. AV-101(4-Chlorokynurenine (4-C1 Kyn) is an orally active small molecule prodrug of 7-chlorokynurenic acid, which acts as an NMDAR glycine-site antagonist.

Other examples of glycine site antagonists that can be used in the pharmaceutical composition and methods described herein are N-(6,7-dichloro-2,3-dioxo-1,2,3,4-tetrahydro-quinoxalin-5-yl)-N-(2-hydroxy -ethyl)-methanesulfonamide and 6,7-dichloro-5-[3-methoxymethyl-5-(1-oxypyridin-3-yl)-[1,2,4]triazol-4-yl]-1,4-dihydro-quinoxaline-2,3-dione.

Additional NMDAR antagonists for use herein are described in Schiene et al., U.S. Pat. Appl. No. US2001/0306674 A1, and include without being limited thereto, N-containing phosphonic acids, such as norvaline (AP5), D-norvaline (D-AP5), 4-(3-phosphono-propyl)-piperazine-2-carboxylic acid (CPP), D-(E)-4-(3-phosphonoprop-2-enyl)piperazine-2-carboxylic acid (D-CPPene), cis-4-(phosphonomethyl)-2-piperidine carboxylic acid (Selfotel, CGS 19755), SDZ-220581, PD-134705, LY-274614 and WAY-126090; quinolinic acids, such as kynurenic acid, 7-chloro-kynurenic acid, 7-chloro-thiokynurenic acid and 5,7-dichloro-kynurenic acid, prodrugs thereof, such as 4-chlorokynurenine and 3-hydroxy-kynurenine; 4-aminotetrahydrochinolin-carboxylates, such as L-689,560; 4-hydroxyquinolin-2(1H)-ones, such as L-701,324; quinoxalinediones, such as licostinel (ACEA-1021) and CGP-68,730A; 4,6-dichloro-indole-2-carboxylate derivatives such as MDL-105,519, gavestinel (GV-150,526) and GV-196,771A; tricyclic compounds, such as ZD-9,379 and MRZ-2/576, (+)-HA-966, morphinan derivatives such as dextromethorphan and dextrophan; benzomorphans, such as BIII-277CL; other opioids, such as dextropropoxyphene, ketobemidone, dextromethadone and D-morphine; amino-adamantanes, such as amantadine and memantine; amino-alkyl-cyclohexanes, such as MRZ-2/579; ifenprodil and ifenprodile-like compounds such as eliprodil and PD-196,860; iminopyrimidines; or other NMDAR-antagonists such as nitroprusside, D-cycloserine, 1-aminocyclopropanecarboxylic acid, dizocilpine (MK 801) and its analogs, phencyclidine (PCP), ketamine ((R,S)-2-(2-Chlorphenyl)-2-(methylamino)cyclohexan-1-on), (R)-ketamine, (S)-ketamine, remacemide and its des-glycinyl-metabolite FPL-12,495, AR-R-15,896, methadone, sulfazocine, AN19/AVex-144, AN2/AVex-73, Besonprodil, CGX-1007, EAB-318, Felbamate and NPS-1407. NMDAR-Antagonists are, for example, disclosed in "Analgesics," edited by H. Buschmann, T. Christoph, E. Friderichs, C. Maul, B. Sundermann, 2002, Wiley-VCH Verlag GmbH & Co. KGaA, Weinheim, Germany, in particular pages 389-428.

Along with identified NMDAR antagonists, additional effective compounds can be identified using well-validated electrophysiological assays such as modulation of NMDAR-mediated responses to NMDAR glutamate-site agonists, or radioreceptor assays, such as modulation of binding to the NMDAR PCP-receptor channel binding site. Glycine site agonists and antagonists can also be distinguished based upon both electrophysiology and receptor binding from compounds such as phencyclidine (PCP) or ketamine that bind to the channel site. Partial agonists are defined as compounds that have reduced efficacy for inducing conformational change in receptors (typically 40-80%) relative to full agonists. Mixed agonists/antagonists are compounds that may induce agonist effects at low dose but antagonist effects at high dose.

The described methods relate to DCS provided in dosages intended to produce blood and/or plasma concentrations above 25 microgram/mL. It will be understood D-cycloserine levels in blood and/or plasma may be determined using standard analytic techniques such as high-pressure liquid chromatography.

Plasma levels associated with anti-depressant effects of D-cycloserine can be determined using rodent behavioral tests such as the forced swim test (FST), which has previously been shown to sensitive to effects of NMDAR glycine site antagonists such as 1-aminocyclopropanecarboxylate ACPC (Trullas et al., Eur J Pharmacol. 1991;203:379-385).

In general, locomotor hyperactivity is considered a rodent analog of psychotomimetic effect. A prior study did not find significant increases in locomotor activity in mice when D-cycloserine was given at doses of up to 160 mg/kg. Nevertheless, D-cycloserine potentiated the effects of a low dose of the non-competitive NMDAR antagonist MK-801 (Carlsson ML, Engberg G, Carlsson A. Effects of D-cycloserine and (+)-HA-966 on the locomotor stimulation induced by NMDARantagonists and clonidine in monoamine-depleted mice. J Neural Transm Gen Sect. 1994;95(3):223-233).

The second of the two active agents in the described pharmaceutical compositions is an antidepression agent.

Said antidepressant can be a tetracyclic antidepressant (TeCA), selective serotonin reuptake inhibitor (SSRI), a serotonin/norephinephrine reuptake inhibitor (SNRI), an antipsychotic approved for treatment of depression or a combination thereof. Other antidepressants for use in the described compositions and methods include monoamine oxidase inhibitors (MAOI), TCAs such as, but not limited to imipramine, amitryptiline, desipramine, clomipramine, TeCAs such as mianserin, mirtazapine, serotonin (SSRI) and serotonin/norephinephrine (SNRI) reuptake inhibitors, such as fluoxetine, fluvoxamine, paroxetine, citalopram, escitalopram, duloxetine, venlafaxine and others, as will be appreciated by the skilled artisan.

Current treatments for major depression consist primarily of older antidepressants, such as monoamine oxidase inhibitors (MAOI) and tricyclic antidepressants (TCAs) (e.g. imipramine, amitryptiline, desipramine, clomipramine) that were first developed in the 1960's, and newer agents such as tetracyclic antidepressants (TeCAs) (e.g., mianserin, mirtazapine), serotonin (SSRI) and serotonin/norephinephrine (SNRI) reuptake inhibitors (e.g., fluoxetine, fluvoxamine, paroxetine, citalopram, escitalopram, duloxetine, venlafaxine, dapoxetine, indalpine, milnacipran, levomilnacipran). MAOIs and TCAs are considered "broader spectrum" agents than SSRIs/SNRIs that were developed subsequently.

Other anti-depressant agents show differential mechanism of action. Bupropion is an antidepressant medication that works as a norepinephrine-dopamine reuptake inhibitor (NDRI) and is also approved for smoking cessation.

SNRIs may differ in relative specificity for serotonin (SERT) vs. norepinephrine (NET) transporters. For example, traditional SNRI agents such as venlafaxine, duloxetine and desvenlafaxine show substantially greater affinity for SERT versus NET transporters, whereas newer agents such as milnacipram and levomilnacipran show more balanced affinity for NET vs. SERT transporters. These compounds thus have serotonin:norepinephrine reuptake ratios more similar to those of TCAs than SSRIs or other SNRIs.

Vilazodone is considered an atypical antidepressant in that it functions as both an SSRI and a partial agonist at 5HT1A receptors and has thus been termed a serotonin partial agonist and reuptake inhibitor (SPARI) (Schwartz et al., Vilazodone: A Brief Pharmacological and Clinical Review of the Novel Serotonin Partial Agonist and Reuptake Inhibitor, Ther Adv Psychopharmacol. 1:81-87, 2011). The combination of serotonin reuptake inhibition and 5-HT1A agonist is believed to function as an antidepressant at lower levels of serotonin transporter occupancy than otherwise (Kohler et al., J Psychopharmacogy, 30:13-22, 2016).

Vortioxetine is considered a multimodal antidepressant in that it functions as 1) a serotonin transport inhibitor, 2) a partial agonist at 5-HT1A receptors, and 3) a partial antagonist of 5-HT1B, 5HT1D and 5-HT7 receptors (Stahl SM, Modes and nodes explain the mechanism of action of vortioxetine, a multimodal agent (MMA): enhancing serotonin release by combining serotonin (SHT) transporter inhibition with actions at SHT receptors (5HT1A, 5HT1B, 5HT1D, SHT7 receptors) CNS Spectrums (2015), 20, 93-97)

Levopmilnacipran and milnacipran, while considered SNRIs, have a much lower ratio of serotonin:norephinephrine ratio than previously marketed SNRI agents. Thus, whereas venlafaxine, duloxetine, and desvenlafaxine all have serotonin:noepinephrine ratios of 10:1 or greater, levomilnacipran has a ratio of only 1.2:1 and milnacipran has a ratio of only 1.6:1 (Sansone RA, Sansone LA Serotonin norepinephrine reuptake inhibitors: a pharmacological comparison. Innov Clin Neurosci. 11 (3-4): 37-42). Binding profiles of milnacipran and levomilnacipran are thus more similar to TCAs than to other SNRIs.

Mirtazapine is a racemic mixture of S(+)mirtazapine (also called S-mirtazapine) and R(-)mirtazapine (also called R-mirtazapine) that functions as a noradrenergic and specific serotonergic antidepressant (NaSSA). Both the the S(+)mirtazapine and R (-) mirtazapine enantiomers are pharmacologically active, but may have offsetting effects. Thus, for example, in one study racemic mirtazapine produced biphasic effects in a rodent nociception assay, whereas R(-)mirtazapine produced only anti-nociceptive effects and S(+)mirtazapine exerted pro-nociceptive effects (Freynhagen et al., Brain Res Bull 69:168-173, 2006). Differences in binding profile are also observed between the enantiomers that confer differential effects on specific aspects of binding, that may confer advantages over the racemate for treatment of specific disorders.

Approved dosing levels for the antidepressant agents described herein can be determined from standard sources, such as packaged inserts approved by the US Food and Drug Administration (FDA).

In some embodiments, the second therapeutic agent is provided at a subtherapeutic dose, if the second therapeutic agent were provided alone.

The provided compositions can be used in methods for treating depression, OCD, and anxiety disorders in a subject in need thereof, in which the subject is administered an oral dose of the described composition as herein described.

In the methods of treating depression, the two active ingredients of the described composition are provided in a single pharmaceutical composition. Alternatively, the active agents are provided separately.

In some embodiments, the regimen comprises a second therapeutic agent which is a psychotropic medication.

In some embodiments, the pharmaceutical compositions can be administered to the patient by any, or a combination, of several routes, for example, whereas D-cycloserine may be administered orally, the second therapeutic agent administered, as herein described may be administered by any appropriate route, for example, such second therapeutic agent may be provided as an oral, intravenous, trans-mucosal (e.g. nasal, vaginal, etc.), pulmonary, transdermal, ocular, buccal, sublingual, intraperitoneal, intrathecal, intramuscular, or long term depot preparation.

In some embodiments, solid compositions for oral administration can contain suitable carriers or excipients, such as corn starch, gelatin, lactose, acacia, sucrose, microcrystalline cellulose, kaolin, mannitol, dicalcium phosphate, calcium carbonate, sodium chloride, lipids, alginic acid, or ingredients for controlled slow release. Disintegrators that can be used include, without limitation, micro-crystalline cellulose, corn starch, sodium starch glycolate and alginic acid. Tablet binders that may be used include, without limitation, acacia, methylcellulose, sodium carboxymethylcellulose, polyvinylpyrrolidone (Povidone), hydroxypropyl methylcellulose, sucrose, starch, and ethylcellulose.

In some embodiments, liquid compositions for oral administration prepared in water or other aqueous vehicles can include solutions, emulsions, syrups, and elixirs containing, together with the active compound(s), wetting agents, sweeteners, coloring agents, and flavoring agents. Various liquid and powder compositions can be prepared by conventional methods for inhalation into the lungs of the patient to be treated.

In some embodiments, the second therapeutic agent may be formulated as an injectable composition, which may contain various carriers such as vegetable oils, dimethylacetamide, dimethylformamide, ethyl lactate, ethyl carbonate, isopropyl myristate, ethanol, polyols (glycerol, propylene glycol, liquid polyethylene glycol, and the like).

In some embodiments, the second therapeutic agent may be formulated as an intravenous injection, the compounds may be administered by the drip method, whereby a pharmaceutical composition containing the active compound(s) and a physiologically acceptable excipient is infused.

Physiologically acceptable excipients may include, for example, 5% dextrose, to 0.9% saline, Ringer's solution or other suitable excipients. For intramuscular preparations, a sterile composition of a suitable soluble salt form of the compound can be dissolved and administered in a pharmaceutical excipient such as Water-for-Injection, 0.9% saline, or 5% glucose solution, or depot forms of the compounds (e.g., decanoate, palmitate, undecylenic, enanthate) can be dissolved in sesame oil. Alternatively, the pharmaceutical composition can be formulated as a chewing gum, lollipop, or the like.

While the dosage regimen and methods described herein represent an optimum arrived at by administering the D-cycloserine orally, it will be appreciated by the skilled artisan that a lower dosage may be accomplished with the same achieved plasma level by administering the D-cycloserine by a route that does not undergo first pass metabolism. According to this aspect, the dosage can be adjusted to be staggered accordingly, as presented for the oral dosage regimens described herein, with proportionately lower dosages, to accommodate a non-oral administration route, and such alterations are to be considered to be an embodied regimen of this invention.

It will also be appreciated by the skilled artisan that a lower dosage may be accomplished with the same achieved plasma level by administering the D-cycloserine in a formulation that undergoes reduced gastric degradation, for example by applying an enteric coating.

In other embodiments, the formulations as herein described, in particular with regard to oral formulations, are envisioned to comprise slow release tablet formulations. Such slow release tablet formulations may, for example, comprise commercially available formulations containing known anti-depressant medications, such as, for example, Effexor (venlafaxine) or Seroquel (quetiapine), both of which are already available in extended length (XR) formulations, however the formulation may be modified to further incorporate D-cycloserine.

In other embodiments, the formulations as herein described, in particular with regard to oral formulations, are envisioned to comprise both short acting and extended release formulations. Extended release formulations have the advantage inter alia of minimizing the difference between peak and trough levels of drug, and thereby to increase effectiveness and/or reduce side effects of a medication.

Methods for the formulation of the described regimens herein are well known, and the skilled artisan will appreciate that it is straightforward to prepare the oral dosage regimens as herein described. Applicants, for example, refer to Gibaldi's Drug Delivery Systems in Pharmaceutical Care, Desai A & Lee M (eds), Bethesda, Md.: American Society of Health-System Pharmacists, 2007.

D-cycloserine has a relatively short half-life in man, and therefore is presently used in BID dosing. In some embodiments of the invention BID dosing is envisioned. According to this aspect, and in some embodiments, such consideration will nonetheless ensure that the daily dosage described for the regimens defined herein are not exceeded.

In some embodiments of the invention, D-cycloserine is microencapsulated to increase its circulating half-life. According to this aspect, and in some embodiments, the microencapsulated compound would then be combined either with an anti-depressant medication that is already administered once daily (e.g. sertraline, citalopram, aripiprazole) to insure that cycloserine cannot be taken without accompanying antidepressant (which would increase risk of CNS side effects). Alternatively, the drug could be combined with an anti-depressant compound that is already typically given in divided doses (e.g. venlafaxine, quetiapine) and the two drugs could then be microencapsulated in common to yield a once-daily formulation with similar half-life between the two ingredients. Microencapsulation using standard approaches for (cf. Doshi DH, Oral Drug Delivery Systems, in Gibaldi's Drug Delivery Systems in Pharmaceutical Care, Desai A & Lee M (eds), Bethesda, Md.: American Society of Health-System Pharmacists, 2007. pp. 23-43) such as use of coating materials or matrix-based oral delivery systems. In one approach, for example, drugs are mixed with a gelling agent, such as hydroxypropylmethylcellulose or hydroxylpropylcellulose, which form a hydrophilic matrix (gel) upon contact with water that delays release of the compound. Release properties can be regulated by selection of specific gelling agents, as is known in the art (see, for example, U.S. Pat. No. 5,948,437; European patent EP20040765928, U.S. Pat. No. 7,807,195).

Other compounds that can be used to control release include cellulose, ethylcellulose, gelatin, hypromellose, iron oxide and titanium oxide. In some matrix systems, drug release is controlled mainly by diffusion through matrix pores and not by the erosion of the polymers. Drug delivery can also be controlled by use of reservoir type systems in which release is controlled by osmotic gradient across the coating membrane. Capsules can be manufactured which contain granules with different microencapsulation properties which can be blended to achieve a composition that has a desired release rate.

In one embodiment of the invention, D-cycloserine is microencapsulated along with quetiapine or a pharmaceutically acceptable salt thereof using a gelling agent such as hydroxypropyl methylcellulose, together with one or more pharmaceutically acceptable excipients. In some embodiments, the sustained release formulation comprises a hydrophilic matrix comprising a gelling agent, preferably hydroxypropyl methylcellulose, D- cycloserine, quetiapine and pharmaceutically acceptable salts thereof, together with one or more pharmaceutically acceptable excipients.

As described, the methods of treatment provided herein include administering to a subject at least two active ingredients for the treatment of depression and other neuropsychiatric conditions.

NMDAR mediate a process termed long-term potentiation that may be excessive in disorders such as depression, obsessive compulsive personality disorder (OCD), post-traumatic stress disorder (PTSD) or other stress-related disorders (SD) and anxiety disorders such as adjustment disorders with depressive, anxious or mixed features. NMDAR excess disorders may be identified by objective biological markers such as reduced brain glutamate + glutamate (Glx) levels as detected using magnetic resonance spectroscopy (MRS) (e.g. Milak et al.,. Mol Psychiatry. 2016;21(3):320-7; Kantrowitz et al.. Am J Psychiatry. 2016;173(12):1241-2).

Major depression is a clinical syndrome that includes a persistent sad mood or loss of interest in activities, which persists for at least two weeks in the absence of treatment. Symptoms of major depression are typically measured using rating scales such as the Hamilton Depression Rating Scale (HAM-D) or the Beck Depression Inventory (BDI). In addition to including symptoms relevant to depressed mood, the HAM-D also contains symptoms sensitive to psychosis, including items for guilt, depersonalization/derealization and paranoia.

Major depression may also be associated with symptoms of anxiety, which may be measured with rating scales such as the Hamilton Rating Scale for Anxiety (HAM-A). Depressive disorders are divided in major depression (MDD) and bipolar depression (BPD), which may be diagnosed using criteria set forth in the Diagnostic and Statistical Manual, 5th edition, published by the American Psychiatric Association (DSM-5), which provides as well additional description of mental disorders. Major depression may also occur with and without melancholic features. In addition, depressive symptoms may occur in the context of anxiety disorders such as generalized anxiety disorder, dissociative disorders, personality disorders or adjustment disorders with depressed mood (DSM-5).

Other forms of depression include atypical depression, agitated depression, depression with mixed emotional features, cyclothymia, dysthymia minor depression and adjustment disorder with depressed mood. Bipolar depression may be divided into Bipolar I and Bipolar II subtypes based upon presence or absence of manic episodes. In bipolar disorder, depressive symptoms can occur in the context of either a depressive episode, or a mixed state in which symptoms of mania and depression occur simultaneously or in rapid sequence. Rapid cycling between mania and depressive episodes may also occur in some individuals.

The first therapeutic agent in the described methods includes an NMDAR antagonist, which is DCS provided at the net antagonist dose described herein. The method further comprises administering a second therapeutic agent for the treatment of depression to said subject. In particular embodiments, the second therapeutic agent is administered in the same composition as the NMDAR antagonist. In other embodiments, it is administered separately.

According to this aspect, the method is not limited in terms of the timing of the administration of the second therapeutic agent, such that the methods of this invention contemplate a subject already treated with a second therapeutic agent, or a naive subject concomitantly treated with D- cycloserine and the second therapeutic agent, or in some embodiments, the subject initially treated with D- cycloserine is then administered a second therapeutic agent, and each of these scenarios represents an embodiment of this invention. According to the invention, the second therapeutic agent is (S)-mirtazapine.

According to this aspect, the method is not limited in terms of the timing of the administration of the second therapeutic agent, such that the methods of this invention contemplate a subject or population already treated with a second therapeutic agent, or a naive subject or population concomitantly treated with D- cycloserine and the second therapeutic agent, or in some embodiments, the subject or population is initially treated with D- cycloserine and then administered a second therapeutic agent, and each of these scenarios represents an embodiment of this invention.

In some embodiments, the invention further provides for the use of D- cycloserine in the preparation of a medicament formulated for oral administration at a dosage of >500 to <1000 mg/day for the treatment of depression in a subject in need thereof, wherein the dose is formulated to produce sustained plasma levels of between 25 - 35 microgram/mL.

In some embodiments, plasma levels produced by D-cycloserine administration are sustained from 30 minutes to 2 hours following administration.

In some embodiments according to the aspect, plasma levels produced by D-cycloserine administration are sustained from 30 minutes to 12 hours following administration.

The following examples are provided to illustrate certain particular features and/or embodiments.

### EXAMPLES

### Reference

### Example 1: Demonstration of locomotor hyperactivity effects of D-cycloserine in rodents and effects of anti-depressant agents

For this study, psychomotor effects of D-cycloserine were assessed using the rodent open field test following D-cycloserine administration, in the presence or absence of antidepressant agents.

All testing was performed at PsychoGenics Inc, 765 Old Saw Mill River Road, Tarrytown, NY 10591, USA

Male C57BL/6J mice (8 weeks old) from Jackson Laboratories (Bar Harbor, Maine) were used. Upon receipt, mice were assigned unique identification numbers (tail marked) and were group housed in OPTImice cages. All animals were acclimated to the colony room for 1 week prior to testing. During the period of acclimation, animals were examined on a regular basis, handled, and weighed to assure adequate health and suitability. Animals were maintained on a 12 /12 light/dark cycle. The room temperature was maintained between 20 and 23°C with a relative humidity maintained between 30% and 70%. Chow and water were provided *ad libitum* for the duration of the study. All testing was performed during the animal's light cycle phase.

Test compounds included
- D-cycloserine (300 mg/kg) was dissolved in PTS vehicle (5%PEG200: 5%Tween80: 90%NaCl) and administered IP at a dose volume of 10 mL/kg in the open field test.
- Bupropion (10 mg/kg) was dissolved in saline and administered IP at a dose volume of 10 mL/kg 30 minutes prior to D-cycloserine in the open field test.
- Desipramine (10 mg/kg) was dissolved in saline and administered IP at a dose volume of 10 mL/kg 30 minutes prior to D-cycloserine in the open field test
- Sertraline (20 mg/kg) was dissolved in sterile water and administered IP at a dose volume of 10 mL/kg 30 minutes prior to D-cycloserine in the open field test
- Venlafaxine (40 mg/kg) was dissolved in saline and administered IP at a dose volume of 10 mL/kg 30 minutes prior to D-cycloserine in the open field test
- Duloxetine (40 mg/kg) was dissolved in saline and administered IP at a dose volume of 10 mL/kg 30 minutes prior to D-cycloserine in the open field test
- Fluoxetine (10 mg/kg) was dissolved in saline and administered IP at a dose volume of 10 mL/kg 30 minutes prior to D-cycloserine in the open field test
- Imipramine (10 mg/kg) was dissolved in saline and administered IP at a dose volume of 10 mL/kg 30 minutes prior to D-cycloserine in the open field test
- Citalopram (10 mg/kg) was dissolved in saline and administered IP at a dose volume of 10 mL/kg 30 minutes prior to D-cycloserine in the open field test
- Levomilnacipran (40 mg/kg) was dissolved in sterile water and administered IP at a dose volume of mL/kg 30 minutes prior to D-cycloserine in the open field test
- Milnacipran (40 mg/kg) was dissolved in sterile water and administered IP at a dose volume of mL/kg 30 minutes prior to D-cycloserine in the open field test
- Vilazodone (1 mg/kg) was dissolved in PTS vehicle (5%PEG200: 5%Tween80: 90%NaCl) and administered IP at a dose volume of 10 mL/kg 30 minutes prior to D-cycloserine in the open field test
- Vortioxetine (10 mg/kg) was dissolved in PTS vehicle (5%PEG200: 5%Tween80: 90%NaCl) and administered IP at a dose volume of 10 mL/kg 30 minutes prior to D-cycloserine in the open field test

The open field (OF) test was performed using Plexiglas square chambers (27.3 x 27.3 x 20.3 cm; Med Associates Inc., St Albans, VT) surrounded by infrared photobeams (16x 16 x 16) to measure horizontal and vertical activities. Mice were brought to the activity experimental room for at least 1 hr acclimation to the experimental room conditions prior to testing. Animals were administered with vehicle, or test compound and placed in the OF. For assessment of D-cycloserine effects, mice were injected with DCS prior to entry into the test chamber, and activity was monitored for 60 min. Alternately, vehicle or D-cycloserine was administered 30-min prior to challenge with amphetamine (4 mg/kg) or phencyclidine (5 mg/kg), and activity was summed for 60 min following vehicle or D-cycloserine administration. For other conditions, animals were treated with vehicle or antidepressant agents, following which baseline activity was recorded for 30 minutes. Mice then received DCS injections and were placed back into OF chambers for a 60 minute session. At the end of each OF test session the OF chambers were thoroughly cleaned.

Data were analyzed by analysis of variance (ANOVA) followed by post-hoc comparisons using Fishers's LSD test as appropriate. An effect was considered significant if p < 0.05.

**Table 1: Summary of Locomotor activity data showing number of animals (N), Mean distance travelled over 30 min (Mean), Standard deviation and statistical comparisons for animals treated with Vehicle or D-cycloserine**

| Condition | N | Mean | Std. Deviation | p vs. Vehicle |
|---|---|---|---|---|
| Vehicle | 10.0 | 4881.4 | 1165.3 | |
| DCS, 30 mg/kg | 10.0 | 4432.1 | 1712.9 | 0.5 |
| DCS, 300 mg/kg | 10.0 | 7588.5 | 1456.2 | <0.001 |
| DCS, 1000 mg/kg | 9.0 | 9049.8 | 1862.9 | <0.001 |

Results: Dose-response was assessed for 30 min after administration of vehicle or D-cycloserine at doses between 30 and 1000 mg/kg. Across all conditions, there was a highly significant effect (F=19.0, df=3,35, p<0.001). Locomotor activity was not significantly affected by D-cycloserine administered at a dose of 30 mg/kg (p=.5), but was significantly increased by both 300 mg/kg (p<.001) and 1000 mg/kg (p<.001) doses of D-cycloserine **(Table 1).**

When conditions were separated according to drug type including NDRIs (buproprion), tricyclic anti-depressants (desipramine, imipramine). SSRIs/SNRIs associated with high serotonergic transport (SERT) inhibition activity (sertraline, venlafaxine, duloxetine, fluoxetine, citalopram) vs. newer agents associated with low serotonergic transport inhibition activity relative to other targets such as norepinephrine transporters or 5-HT1A receptors (levomilnacipran, milnacipran, vilazodone, vortioxetine), unexpected differences among the drug classes were observed **(Table 2).**

Across all medication types, there was a highly significant main effect of treatment type (F=12.5, df=3,116, p<0.001). Among antidepressants, highest levels of activity (greatest psychotomimetic effect) were observed with DCS combined with either traditional SSRIs/SNRIs or bupropion. By contrast, locomotor activity was significantly lower with DCS combined with TCAs (p<0.001) or newer SNRIs/atypical antidepressants (p<0.001) relative to traditional SSRIs/SNRIs. In addition, locomotor activity was significantly lower in combination with milnacipran/levomilnacipran (p=0.003), vilazodone (p=0.007) and vortioxetine (p=0.014) than with traditional SSRIs/SNRIs, suggesting significant individual utility of these agents in combination with D-cycloserine.

**Table 2: Summary of locomotor activity data showing number of animals (N), Mean distance travelled over 60 min (Mean), Standard deviation and statistical comparisons for animals treated with Vehicle or D-cycloserine (DCS)**

| **Condition (DCS 300 mg/kg + indicated agents)** | **N** | **Mean** | **Std. Deviation** | **p val vs. traditional SSRIs/SNRIs** |
|---|---|---|---|---|
| **Traditional SSRIs/SNRIs** | 50 | 11307.45 | 8447.01 | --- |
| **bupropion** | 10 | 11526.89 | 6781.38 | p=0.9 |
| **TCAs** | 20 | 2210.92 | 2216.64 | p<0.001 |
| **milnacipran/levomilnacipran** | 20 | 5996.44 | 4680.51 | p=0.003 |
| **vilazodone** | 10 | 5118.55 | 3008.78 | p=0.007 |
| **vortioxetine** | 10 | 5682.96 | 6096.93 | p=0.014 |

This is the first study of which we are aware to show that D-cycloserine on its own produces locomotor hyperactivity in rodents, suggestive of a clinical psychotomimetic effect. Taken together with a prior study showing no significant effect of D-cycloserine administered at a dose of 160 mg/kg (Carlsson et al., J Neural Transm 95:223-233, 1994), these findings demonstrate that psychotomimetic effects are observed preferentially at plasma levels exceeding 25 micrograms/mL (see Example 3).

This is also the first study of which we are aware to show that antidepressants show differential effects on locomotor hyperactivity in the presence of D-cycloserine, with preferential effect noted for agents such as TCAs or the newer antidepressants vilazodone, vortioxetine, milnacipran and levomilnacipran, relative to traditional SSRI/SNRIs or bupropion, a dopamine-norepinephrine reuptake inhibitor.

Newer SNRIs and atypical antidepressants differ from traditional medications in that they have higher specificity for targets other than the serotonin transporter, and thus are relatively more similar to TCAs. These findings show unexpectedly that newer SNRIs/atypical antipsychotics have preferential beneficial activities in combination with a D-cycloserine-dose associated with plasma levels >25 microgram/mL, and thus show unexpected utility of combinations involving high dose D-cycloserine and newer anti-depressant agents.

### Reference

### Example 2: Effects of NMDAR antagonists alone and combined with antidepressants and antidepressants in the rodent forced swim test

For this study, anti-depressant effects of NMDAR antagonists were assessed using the rodent forced swim tests. NMDAR antagonists were studied alone and in combination with specific antidepression agents.

All testing was performed at PsychoGenics Inc, 765 Old Saw Mill River Road, Tarrytown, NY 10591, USA

Male BalbC/J mice (8 weeks old) from Jackson Laboratories (Bar Harbor, Maine) were used. Upon receipt, mice were assigned unique identification numbers (tail marked) and were group housed in OPTImice cages. All animals were acclimated to the colony room for 1 week prior to testing. During the period of acclimation, animals were examined on a regular basis, handled, and weighed to assure adequate health and suitability. Animals were maintained on a 12 /12 light/dark cycle. The room temperature was maintained between 20 and 23°C with a relative humidity maintained between 30% and 70%. Chow and water were provided ad libitum for the duration of the study. All testing was performed during the animal's light cycle phase.

Mice were acclimated to the test room at least 1 hour prior to commencing the test. The forced swimming test consisted of one 6-minute session of forced swimming in individual opaque cylinders (15 cm tall x 10 cm wide, 1000 ml beakers) containing fresh tap water at a temperature of 23 ± 2 °C and a depth of 12 cm (approximately 800 ml) for each test animal. The time the animal spent immobile was recorded over the 6 min trial. Every 1 min the cumulative immobility time was recorded from the start of the session and noted on the study data record sheet. Immobility was defined as the postural position of floating in the water. The animals were generally observed with the back slightly hunched and the head above water with no movements or with small stabilizing movements of the limbs. After the swim test, each animal was placed in a pre-heated cage with a heating pad and allowed to dry.

The main variable used to represent immobility in this test was the total time immobile during the 6-min test period. Statistics were performed by analysis of variance (ANOVA) with Dunnett post-hoc testing vs. the control condition or by t-test as appropriate.

NRIs, SSRIs, SNRIs, TeCAs, atypical and multimodal antidepressants are all indicated for the treatment of depression. In this example, potential for additive effect was evaluated across a range of anti-depressants. The forced swim test was used as described in Example 1. Compounds tested were as follows:
- D-cycloserine (DCS 300 mg/kg) was dissolved in PTS vehicle and administered IP at a dose volume of 10 ml/kg 30 min prior to test.
- Bupropion (10 mg/kg) was dissolved in saline and administered IP at a dose volume of 10 mL/kg 30 minutes prior to test.
- Desipramine (10 mg/kg) was dissolved in saline and administered IP at a dose volume of 10 mL/kg 30 minutes prior to test.
- Imipramine (10 mg/kg) was dissolved in saline and administered IP at a dose volume of 10 mL/kg 30 minutes prior to test.
- Sertraline (20 mg/kg) was dissolved in saline and administered IP at a dose volume of 10 mL/kg 30 minutes prior to test.
- Venlafaxine (40 mg/kg) was dissolved in saline and administered IP at a dose volume of 10 mL/kg 30 minutes prior to test.
- Duloxetine (40 mg/kg) was dissolved in saline and administered IP at a dose volume of 10 mL/kg 30 minutes prior to test.
- Fluoxetine (10 mg/kg) was dissolved in saline and administered IP at a dose volume of 10 mL/kg 30 minutes prior to test.
- Citalopram (10 mg/kg) was dissolved in saline and administered IP at a dose volume of 10 mL/kg 30 minutes prior to test.
- Levomilnacipran (40 mg/kg) was dissolved in sterile water and administered IP at a dose volume of mL/kg 30 minutes prior to test
- Milnacipran (40 mg/kg) was dissolved in sterile water and administered IP at a dose volume of mL/kg 30 minutes prior to test
- Vilazodone (1 mg/kg) was dissolved in PTS vehicle and administered IP at a dose volume of 10 mL/kg 30 minutes prior to test.
- Vortioxetine (10 mg/kg) was dissolved in PTS vehicle and administered IP at a dose volume of 10 mL/kg 30 minutes prior to test.
- Mirtazapine (5 mg/kg) were dissolved in PTS vehicle and administered IP at a dose volume of 10 mL/kg 30 minutes prior to test.

Results are shown in **Figure 2****.** Effects of DCS 300 mg/kg were highly significant across all agents tested. Particular beneficial effects were observed for bupropion, sertraline, and duloxetine where effects of combined antidepressant and DCS 300 mg/kg were significantly larger than with either agent alone.

Results for D-cycloserine (DCS) alone are shown in **Figure 1****.** DCS had no significant effect in the forced swim test assay at a dose of 30 mg/kg. By contrast, DCS had a statistically significant (p<.001) reduction when administered at doses of 100 mg/kg or above.

Results for combined DCS and antidepression agents are shown in **Figure 2****.** In all cases, effects of DCS were maintained in the presence of specified agents, as reflected in the difference between combined treatment and the control, no DCS condition. Specific addition beneficial effects were observed in combination with bupropion, TCAs, sertraline, venlafaxine, duloxetine, fluoxetine, vortioxetine and mirtazapine. Combinations with bupropion, sertraline, and duloxetine produced effects greater than DCS alone.

### Example 3: Pharmacokinetics of D-cycloserine in rodents

For this study, the pharmacokinetics of D-cycloserine in rodents were assessed. This study tests the hypothesis that antidepressant effects of DCS presented in the above examples are observed specifically at treatment levels that produce sustained blood DCS levels of >25 microgram/mL.

For this study, male C57BL/6J mice (8 weeks old) from Jackson Laboratories (Bar Harbor, Maine) were used. D-cycloserine (30, 100, 300, 500 and 1000 mg/kg) was dissolved in PTS vehicle (5%PEG200: 5%Tween80: 90%NaCl) and administered IP at a dose volume of 10 mL/kg.

For each treatment group a total of 12 mice were dosed: 4 mice were collected at 30, 60 and 120 min. Mean plasma level was computed over the 30-60 min time period.

Analysis of DCS in plasma was performed utilizing an UPLC/MS/MS system consisted of an Acquity UPLC chromatographic system and a Quattro Premier XE triple quad mass spectrometer, both from Waters. Isolation of DCS was achieved using a 5 minute (total run time) HILIC methodology which provided an LLOQ of 5ng/mL.

Results of the experiment are shown in **Table 1.** As shown, peripheral D-cycloserine administration was associated with a dose-dependent increase in plasma D-cycloserine over the 30-60 min time period (p<.0001). Plasma levels associated with 30 mg/kg DCS treatment were associated with a mean plasma level below 25 microgram/mL. Plasma levels associated with the 100 mg/kg dose or higher were all significantly different from 25 µg/mL (p<.001). The maximum tolerated dose was 500-1000 mg/kg, suggesting a maximum tolerated blood level of approximately 500 ug/mL.

These findings provide a demonstration that blood D-cycloserine levels associated with an antidepression effect in rodents (300 mg/kg) produce plasma levels in excess of 25 microgram/mL.

**Table 3: Mean plasma levels (microgram/mL) over 30-60 min following administration of D-cycloserine to rodents**

| Dose (mg/kg) | mean | Std dev | Std err | p-value vs. 25 µg/mL |
|---|---|---|---|---|
| 30 | 24.89 | 4.86 | 2.43 | 0.966 |
| 100 | 74.92 | 7.26 | 3.63 | <0.001 |
| 300 | 251.48 | 28.67 | 14.34 | <0.001 |
| 500 | 375.44 | 25.20 | 12.60 | <0.000 |
| 1000 | 510.73 | 71.56 | 35.78 | <0.001 |

### Example 4: Antidepressant and anti-anxiety effects of combined DCS + anti-depressants

As noted in Example 1, significant beneficial effects were observed with the combination of mirtazapine + D-cycloserine. Mirtazapine is a racemic compound consisting of 2 stereoisomers: R-ketamine and S-ketamine. Here, we evaluated the relative effects of the isomers on forced swim test activity.

Test compounds included DCS formulated as described above, and Mirtazapine, R- Mirtazapine and S- Mirtazapine (5 mg/kg) which were dissolved in PTS vehicle and administered IP at a dose volume of 10 mL/kg 30 minutes prior to test.

Results are shown in **Table 4:** As shown, highly significant beneficial effects were observed with all combinations as shown by highly significant p-values vs. control. In all combinations, DCS also added significantly to anti-depressant effects of mirtazapine or its isomers as shown by significant p values for DCS vs. no DCS. Finally, significantly greater beneficial improvement was noted with combined DCS + R-mirtazapine vs. DCS + S-mirtazapine as shown by the significant values between the two compounds. Although R-mirtazapine and racemic mirtazapine did not differ statistically, reduction in immobility was numerically greater in the presence of R- vs. racemic mirtazapine in both the absence and presence of DCS.

These studies demonstrate unanticipated preferential effects of R-mirtazapine + DCS vs. other combinations.

**Table 4: Immobility time (sec) in the rodent forced swim test (FST) under indicated conditions.**

| Condition | N | Mean | Std | p-value vs. ctl | p-value: DCS vs. no DCS | p-value: R vs. S-mirtazapine |
|---|---|---|---|---|---|---|
| Ctl | 50 | 127.50 | 42.97 | --- | --- | |
| DCS 300 mg/kg | 50 | 42.08 | 47.60 | p<0.001 | p<0.001 | |
| Mirtzapine 5 mg/kg | 10 | 92.30 | 66.59 | p=0.03 | --- | |
| Mirtzapine + DCS | 10 | 31.20 | 26.47 | p<0.001 | p<0.001 | |
| S-mirtazipine 5 mg/kg | 10 | 117.60 | 43.44 | p<0.001 | --- | |
| S-mirtazipine + DCS | 10 | 72.50 | 48.04 | p<0.001 | p<0.001 | |
| R-mirtazipine 5 mg/kg | 110 | 68.40 | 42.64 | p<0.001 | --- | p=0.016 |
| R-mirtazipine + DCS | 110 | 27.60 | 32.80 | p<0.001 | p<0.001 | p=0.028 |

from the cages and the number of unburied marbles was counted. A marble was considered buried if it was covered at least two thirds with bedding.

The following compounds were used. All compounds were administered at a dose volume of 10 ml/kg:
- D-cycloserine (Sigma, DSC; 30 and 300 mg/kg) was dissolved in 5% PEG 200: 5%Tween 80: 90% saline (PTS) and administered IP 30 min prior to test at a dose volume of 10 ml/kg.
- Mirtazapine *(Sigma,* 5.5 mg/kg) was dissolved in 5% PEG 200: 5%Tween 80: 90% saline (PTS) and administered IP 30 min prior to test at a dose volume of 10 ml/kg
- Paroxetine *(Sigma,* 5 mg/kg) was used as the positive reference in the marble burying test. This compound was dissolved in 20% cyclodextrin and administered IP 30 min prior to test at a dose volume of 10 ml/kg.
- Combination DSC (300 mg/kg) +Mirtazapine (5.5 mg) was administered IP as a cocktail in a single injection 30 minutes prior to test at a dose volume of 10 ml/kg.

10 mice were tested in each of the following test groups:
- Vehicle (5% PEG200; 5%Tween80; 90% Saline)
- Paroxetine (5 mg/kg)
- Mirtazapine (5.5 mg/kg)
- D-cycloserine (30 mg/kg)
- D-cycloserine (300 mg/kg)
- D-cycloserine (300 mg/kg) + (Mirtazapine 5.5 mg/kg)

Results of the study are shown in Fig. 3. One-Way ANOVA found a significant treatment effect. Post-hoc comparisons demonstrated that Paroxetine (5 mg/kg) as well as Mirtazapine (5.5 mg/kg) significantly reduced the number of marbles buried compared to vehicle. Treatment of animals with either dose of D-Cycloserine (30 mg/kg, and 300 mg/kg) did not affect this measure. The combination of D-cycloserine (300 mg/kg) and Mirtazapine (5.5 mg/kg) significantly reduced the number of marbles buried compared to vehicle and Mirtazapine (5.5 mg/kg) alone.

These findings demonstrate significant unexpected synergy between DCS, administered at an NMDAR antagonist, 300 mg/kg dose, and the anti-depressant mirtazapine on behaviors related to anxiety, OCD and PTSD, and support combined NMDAR antagonist and anti-depressant treatment of PTSD.

### Example 6: Differential effects of R- and S-isomers of mirtazapine on marble burying

Mirtazapine is a racemic mix of separate R(-) and S(+) isomers. A follow-up study evaluated the relative effects of the two isomers independently. Methods are the same as for Example 3. Test compounds are as follows:
- 5% PEG 200: 5%Tween 80: 90% saline (PTS) was administered IP 30 min prior to test at a dose volume of 10 ml/kg
- Paroxetine (5 mg/kg) was dissolved in saline and was administered IP 30 min prior to test at a dose volume of 10 ml/kg
- D-cycloserine *(Sigma,* DSC; 300 mg/kg) was dissolved in 5% PEG 200: 5%Tween 80: 90% saline (PTS) and administered IP 30 min prior to test at a dose volume of 10 ml/kg.
- Mirtazapine *(Sigma,* 1,2.5, 5.0, and 10 mg/kg) was dissolved in 5% PEG 200: 5%Tween 80: 90% saline (PTS) and administered IP 30 min prior to test at a dose volume of 10 ml/kg
- S-Mirtazapine *(TRC, 1,2.5,* 5.0, and 10 mg/kg) was dissolved in 5% PEG 200: 5%Tween 80: 90% saline (PTS) and administered IP 30 min prior to test at a dose volume of 10 ml/kg
- R-Mirtazapine *(TRC, 1,2.5,* 5.0, and 10 mg/kg) was dissolved in 5% PEG 200: 5%Tween 80: 90% saline (PTS) and administered IP 30 min prior to test at a dose volume of 10 ml/kg

The effects of paroxetine, mirtazapine, S-mirtazapine, R-mirtazapine and D-cycloserine on marble-burying behavior are presented in Figure 4. One-Way ANOVA found a significant treatment effect. Post-hoc comparisons demonstrated that paroxetine (5 mg/kg), mirtazapine (1, 2.5, 5, and 10 mg/kg), S-mirtazapine (1, 2.5, 5, 10 mg/kg), as well as R-mirtazapine (10 mg/kg) significantly reduced the number of marbles buried compared to vehicle. R-mirtazapine (1, 2.5, and 5 mg/kg), did not affect this measure. Effects of S-mirtazapine were significantly more robust than racemic mirtazapine, whereas effects of R-mirtazapine were less robust **(****Fig. 2****),** showing superiority of S-isomer over the racemate for treatment of anxiety-related conditions including OCD and PTSD.

Across the 3 mirtazapine formulations (racemate, R-, S-) there was a highly significant main effect of DCS treatment (F=27.2, df=1,54, p<0.001) supporting prior findings. Mirtazapine (2.5 mg/kg) + DSC (300 mg/kg) combination significantly reduced number of marbles buried compared to mirtazapine (2.5 mg/kg) alone (p<0.01). Additionally, the combination of R-mirtazapine (2.5 mg/kg) + DSC (300 mg/kg) significantly reduced number of marbles buried compared to combination of R-mirtazapine (2.5 mg/kg) + PTS vehicle (p<0.01). In the presence of S-mirtazapine, floor level effects were observed in both the absence and presence of DCS, so no comparisons could be performed.

## Claims

1. A composition for use in treatment of a NMDA receptor neuropsychiatric disorder in a human subject in need thereof, comprising a therapeutic effective amount of:
a first agent consisting of D-cycloserine or a pharmaceutically acceptable salt thereof, provided at a net antagonistic dose of ≥500 mg/day to ≤1000 mg/day and is formulated to produce an average plasma level in the subject of greater than 25 µg/mL; and
a second agent consisting of S-mirtazapine,
and wherein the NMDA receptor neuropsychiatric disorder is selected from the group consisting of obsessive compulsive disorder and anxiety disorders.

2. The composition for use of claim 1, wherein the D-cycloserine is administered at a dose of 7.5-12.5 mg/kg/day.

## Patentansprüche

1. Zusammensetzung zur Verwendung bei der Behandlung einer NMDA-Rezeptorbedingten neuropsychiatrischen Erkrankung bei einem behandlungsbedürftigen menschlichen Individuum, umfassend eine therapeutisch wirksame Menge an:
einem ersten Wirkstoff, bestehend aus D-Cycloserin oder einem pharmazeutisch akzeptablen Salz davon, der mit einer Nettodosis des Antagonisten von ≥500 mg/Tag bis ≤1000 mg/Tag bereitgestellt wird und dazu formuliert ist, in dem Individuum einen durchschnittlichen Plasmaspiegel von mehr als 25 µg/ml zu bewirken; und
einem zweiten Wirkstoff, der aus S-Mirtazapin besteht,
und wobei die NMDA-Rezeptor-bedingte neuropsychiatrische Erkrankung aus der Gruppe bestehend aus Zwangsstörung und Angststörungen ausgewählt ist.

2. Zusammensetzung zur Verwendung nach Anspruch 1, wobei das D-Cycloserin in einer Dosis von 7,5-12,5 mg/kg/Tag verabreicht wird.

## Revendications

1. Composition pour l'utilisation dans le traitement d'un trouble neuropsychiatrique lié aux récepteurs NMDA dans un sujet humain nécessitant un traitement, comprenant une quantité thérapeutique efficace :
d'une première agente consistant en D-cyclosérine ou un sel pharmaceutiquement acceptable du lequel, prévu à une dose nette d'antagoniste de ≥500 mg/jour à ≤1000 mg/jour et qui est formulée pour produire dans le sujet un niveau moyen de plasma supérieur de 25 µg/mL; et
d'une deuxième agente consistant en S-mirtazapine,
et le trouble neuropsychiatrique lié aux récepteurs NMDA étant choisi parmi le groupe consistant en le trouble obsessionnel compulsif et les troubles anxieux.

2. Composition pour l'utilisation de la revendication 1, la D-cyclosérine étant administrée à une dose de 7.5-12.5 mg/kg/jour.
